# EUROPÄISCHE PATENTSCHRIFT

(11) **EP 4 003 226 B1**
(45) Veröffentlichungstag und Bekanntmachung des Hinweises auf die Patenterteilung: **14.06.2023**
(21) Anmeldenummer: 20739617.7
(22) Anmeldetag: 08.07.2020
(51) Int. Cl.: A61F 2/16

(54) **INTRAOKULARLINSE MIT SPREIZBARER HAPTIK**
INTRAOCULAR LENS WITH A SPREADABLE HAPTIC
LENTILLE INTRAOCULAIRE AYANT UN HAPTIQUE POUVANT S'ÉTALER

(30) Priorität: 31.07.2019 DE 102019211435
(43) Veröffentlichungstag der Anmeldung: 01.06.2022
(73) Patentinhaber: Carl Zeiss Meditec AG, 07745 Jena (DE)
(72) Erfinder: KIRCHNER, Friedrich, 10559 Berlin (DE)
(74) Vertreter: Carl Zeiss AG - Patentabteilung
(86) Internationale Anmeldenummer: PCT/EP2020/069253
(87) Internationale Veröffentlichungsnummer: WO 2021/018533

(56) Entgegenhaltungen:
- WO-A1-03/015668
- DE-A1-102007 057 122

## Beschreibung

### Technisches Gebiet

Die Erfindung betrifft eine Intraokularlinse mit einem Optikkörper und mit zumindest einer Haptik, die mit dem Optikkörper gekoppelt ist. Die Intraokularlinse weist eine optische Hauptachse auf, die eine Vorderseite und eine Rückseite des Optikkörpers durchdringt. Diese zumindest eine Haptik weist ein erstes Haptikteil und zumindest ein benachbart dazu ausgebildetes zweites Haptikteil auf. Das zweite Haptikteil ist relativ zum ersten Haptikteil elastisch bewegbar.

### Stand der Technik

Intraokularlinsen sind in vielfältigen Ausgestaltungen bekannt. Üblicherweise weisen Intraokularlinsen zumindest zwei separate Haptiken auf, die in Umlaufrichtung um die optische Hauptachse gegenüberliegend ausgebildet sind und mit dem Optikkörper gekoppelt sind. Es können auch mehr als zwei derartige separate Haptiken ausgebildet sein, beispielsweise drei Haptiken.

Intraokularlinsen können an unterschiedlichen definierten Positionen im Auge anstelle einer natürlichen Linse des Auges implantiert werden. So ist es in dem Zusammenhang vorgesehen, dass spezifische Intraokularlinsen in einer Vorderkammer des Auges implantiert werden. Beispielsweise können derartige Vorderkammerlinsen im vorderen Kammerwinkel fixiert werden.

Darüber hinaus sind Intraokularlinsen bekannt, die als Iris-Clip-Linsen bezeichnet werden. Derartige Intraokularlinsen werden an der Pupille befestigt. Insbesondere werden sie dabei an die Pupillenöffnung geklemmt. Eine derartige Intraokularlinse ist beispielsweise aus der DE 10 2007 057 122 A1 bekannt. Die dortige Intraokularlinse mit diesem spezifischen Implantationsort im Auge weist zwei gegenüberliegende Haptiken auf. Jede dieser Haptiken weist zwei C-förmig geformte Haptikarme auf. Einander zugewandte Enden dieser Haptikarme sind, in einer Ebene senkrecht zur optischen Hauptachse dieser Intraokularlinse betrachtet, einander zugewandt angeordnet, jedoch berührungslos und überlappungsfrei angeordnet. Mittels derartig gebildeter Haptikarme kann durch den gebildeten Spalt, der in Umlaufrichtung um die optische Hauptachse zwischen den Enden der Haptikarme gebildet ist, die Iris eingeklemmt werden. Derartige Linsen sind jedoch nicht dazu vorgesehen und tauglich, in einem Kapselsack eines Auges implantiert werden zu können.

Diesbezüglich sind weitere spezifische Intraokularlinsen bekannt, die als Hinterkammerlinsen bezeichnet werden können und in einen Kapselsack des Auges implantiert werden.

Aus der DE 103 10 961 B4 ist eine Intraokularlinse bekannt, die eine Hinterkammerlinse ist. Bei dieser Hinterkammerlinse ist vorgesehen, dass zwei separate Haptiken an gegenüberliegenden Bereichen des Optikkörpers radial anschließend an den Optikkörper ausgebildet sind. Beide jeweiligen Haptiken sind mit zwei haptischen Teilen ausgebildet. Die beiden haptischen Teile einer Haptik sind relativ zueinander bewegbar. Dazu ist an einer definierten Verbindungsstelle zwischen den einstückig miteinander verbundenen Haptikteilen eine definierte Knickstelle, beispielsweise als Filmscharnier, ausgebildet. Dadurch kann das radial äußere haptische Teil dieser Haptik gegenüber dem ersten haptischen Teil, welches direkt an dem Optikkörper anschließt, geknickt beziehungsweise geschwenkt werden. Diese Schwenkbewegung ist nur in der Ebene senkrecht zur optischen Achse möglich. Dadurch soll dort die radiale Weite der gesamten Intraokularlinse reduziert werden, um Reizungen im Inneren des Kapselsacks durch diese Haptiken vermeiden zu können.

Ferner ist in US 3 994 027 A, DE 10 2007 057122 A1 und WO 03/045668 A1 jeweils eine Intraokularlinse mit mindestens zwei Haptiken beschrieben.

Nach einer Implantation einer Intraokularlinse tritt eine Sehverbesserung auf, welche jedoch innerhalb von 5 Jahren nach der Operation bei etwa 45 % der über 60-jährigen Patienten wieder deutlich nachlässt. Die Ursache sind residuale Linsenepithelzellen auf einer Innenseite des Kapselsackes, die nach einer Katarakt-Operation verbleiben. Durch Proliferation, Migration, Epithelial-mesenchymale Transition (EMT), Kollagenveränderungen und Regeneration von Linsenfasern aus Linsenepithelzellen kommt es zu einer zunehmenden Trübung der hinteren Kapselsackwand, auch "Nachstar" oder "posterior capsule opacification" oder "PCO" genannt. Eine solche Trübung wird für die Patienten als sehr störend empfunden.

### Darstellung der Erfindung

Es ist eine Aufgabe der vorliegenden Erfindung, eine Intraokularlinse zu schaffen, mit der sich die Wahrscheinlichkeit oder Intensität für einen Nachstar reduzieren lässt.

Diese Aufgabe wird durch eine künstliche Intraokularlinse gemäß den Merkmalen des unabhängigen Anspruchs gelöst.

Ein Aspekt der Erfindung betrifft eine Intraokularlinse mit einem Optikkörper und mit zumindest einer Haptik, die mit dem Optikkörper gekoppelt ist. Die Intraokularlinse weist eine optische Hauptachse auf, die eine Vorderseite und eine Rückseite des Optikkörpers durchdringt. Die Haptik weist ein erstes Haptikteil und zumindest ein benachbart dazu ausgebildetes zweites Haptikteil auf, welches relativ zum ersten Haptikteil elastisch bewegbar ist. Die Haptik weist in der Richtung der optischen Hauptachse gemessen maximal eine erste Höhe auf. Dies bedeutet, dass diese erste Höhe nicht entlang der gesamten Erstreckung der Haptik in radialer Richtung ausgebildet sein muss. Vielmehr reicht es aus, wenn eine derartige Höhe zumindest an einer Stelle entlang der Erstreckung in radialer Richtung vorliegt. Das zweite Haptikteil ist relativ zum ersten Haptikteil von einer nicht gespreizten Stellung in eine Spreizstellung so verlagerbar, dass die Haptik in der Richtung der optischen Hauptachse gemessen maximal eine zweite Höhe erreicht, welche größer als die erste Höhe ist. Die erste Höhe ist daher an jeder Stelle der Haptik kleiner als die zweite Höhe. Die zweite Höhe kann daher nur in der Spreizstellung der beiden Haptikteile zueinander vorliegen. Außerdem ist in der Spreizstellung das zweite Haptikteil relativ zu dem ersten Haptikteil arretierbar.

Gemäß der Erfindung weist das erste Haptikelement zumindest ein erstes Spreizelement auf, und das zweite Haptikelement weist zumindest ein zweites Spreizelement auf. Diese beiden Spreizelemente sind in der nicht gespreizten Stellung der Haptikteile direkt aneinander anliegend angeordnet. Sie können somit auch in der nicht gespreizten Stellung berührend zueinander positioniert sein.

Gemäß der Erfindung ist vorgesehen, dass das erste Spreizelement und das zweite Spreizelement zumindest in der Spreizstellung in Umlaufrichtung um die optische Hauptachse betrachtet überlappend zueinander angeordnet sind. Sie sind in dieser Spreizstellung zumindest bereichsweise direkt aneinander anliegend angeordnet.

Durch eine derartige Ausgestaltung der Intraokularlinse ist es ermöglicht, dass mittels des ersten Haptikteils und des zweiten Haptikteils zumindest zwei definierte Stellungen erzielt werden können. Insbesondere ist die Spreizstellung dazu vorgesehen, dass im implantierten Zustand der Intraokularlinse in einem Kapselsack eine geringere Fläche einer Kapselsackwand, insbesondere der hinteren Kapselsackwand, mit einer Rückseite des Optikkörpers in Kontakt kommt. Damit kann Kammerwasser z.B. zwischen diese Rückseite des Optikkörpers und der hinteren Kapselsackwand gelangen, sodass einige der störenden Zellen dort nicht verbleiben, sondern fortgespült werden können. Dies ist vorteilhaft, da somit eine Eintrübung der hinteren Kapselsackwand und damit die Bildung von Nachstar zumindest im Vergleich zu herkömmlichen kapselsackimplantierten Intraokularlinsen verringert werden kann.

Indem das erste Spreizelement und das zweite Spreizelement in der Spreizstellung in Umlaufrichtung um die optische Hauptachse betrachtet überlappend zueinander angeordnet und zumindest bereichsweise direkt aneinander anliegend angeordnet sind, wird die diskrete Spreizstellung, bei welcher die beiden Spreizelemente und somit auch die Haptikteile in der Richtung der optischen Hauptachse gegeneinander verstellt sind und diesbezüglich ein Aufspreizen der Haptikteile ausgebildet ist, besonders vorteilhaft erreicht. Insbesondere ist dadurch auch eine entsprechende mechanische Stabilität bezüglich der Aufrechterhaltung der Spreizstellung erreicht.

Es wird darauf hingewiesen, dass in dieser Schrift die vordere Kapselsackwand zur Hornhaut orientiert ist, wohingegen die hintere Kapselsackwand zur Retina orientiert ist.

In der Spreizstellung bilden das erste Haptikteil und das zweite Haptikteil eine Spreizverbindung. Da das erste Haptikteil und das zweite Haptikteil in der Spreizstellung arretierbar sind, kann diese Spreizstellung dauerhaft beibehalten werden. Ein selbstständiges Rückführen der beiden Haptikteile von der Spreizstellung in die nicht gespreizte Stellung ist verhindert. Insbesondere ist diese Spreizverbindung entsprechend dazu ausgebildet.

Unter einer Spreizverbindung wird eine Verbindung verstanden, bei der mindestens ein zu verformendes Bauteil verformt und gefügt wird und in seiner Endstellung mit geringer Spannung verbleibt. Mindestens in einem Teilbereich steht dieses Bauteil unter permanenter Verformung, wenn es sich in der Spreizstellung befindet.

Insbesondere ist die Intraokularlinse einstückig ausgebildet.

In einer vorteilhaften Ausführung ist die zweite Höhe zumindest derart, dass eine in vertikaler Richtung oder in der Richtung der optischen Hauptachse bemessene maximale Höhe des Optikkörpers kleiner als diese zweite Höhe ist. Somit ist diese zweite Höhe größer als eine in der Richtung der optischen Hauptachse bemessene maximale Dicke des Optikkörpers. Es kann sein, dass die maximale Dicke bzw. Höhe des Optikkörpers in der geometrischen Mitte des Optikkörpers angeordnet ist. In diesem Fall kann die maximale Dicke des Optikkörpers als Mittendicke bezeichnet werden.

Durch dieses Ausführungsbeispiel ist auch sichergestellt, dass es keinen Kontakt zwischen einer Seite des Optikkörpers der Intraokularlinse und einer Wand des unter Spannung stehenden Kapselsackes gibt. Insbesondere ist somit ein direkter Kontakt zwischen der Rückseite des Optikkörpers und der hinteren Kapselsackwand vermieden. Wenn die Spreizstellung dazu führt, dass sich nach einer Implantation der Intraokularlinse die gesamte hintere Kapselsackwand in einem Abstand zur Rückseite des Optikkörpers befindet, ist ein noch besseres Umspülen des Optikkörpers mit Kammerwasser möglich, sodass sich die Wahrscheinlichkeit für die Bildung eines Nachstars weiter reduzieren lässt.

In einer vorteilhaften Ausführung ist vorgesehen, dass die zumindest zwei Haptikteile der zumindest einen Haptik in der nicht gespreizten Stellung der Haptikteile und somit in einer Grundstellung der Haptikteile in einer gemeinsamen Ebene angeordnet sind, die senkrecht zur optischen Hauptachse orientiert ist. Insbesondere stehen diese Haptikteile dann in dieser nicht gespreizten Stellung sowohl in die eine Richtung der optischen Hauptachse als auch in die andere Richtung der optischen Hauptachse nicht gegeneinander über.

In der Spreizstellung ist mindestens eines der beiden Haptikteile so positioniert, dass es aus dieser gemeinsamen Ebene herausragt. Bevorzugt ragen in der Spreizstellung sowohl das erste Haptikteil als auch das zweite Haptikteil aus der Ebene heraus, in welcher sich die beiden Haptikteile im nicht gespreizten Zustand befinden.

Vorzugsweise wird die erste Höhe an der dicksten Stelle der Haptik in der nicht gespreizten Stellung und die zweite Höhe an der dicksten Stelle der Haptik in der Spreizstellung betrachtet.

Insbesondere sind die zumindest zwei relativ zueinander bewegbaren Haptikteile der zumindest einen Haptik in Umlaufrichtung um die optische Hauptachse, also in azimutaler Richtung, benachbart zueinander angeordnet, bevorzugt direkt aneinander anschließend angeordnet. Gemäß einer Ausführungsform können sie sich sowohl in der nicht gespreizten Stellung als auch in der zumindest einen Spreizstellung berühren. Die mindestens zwei Haptikteile können aber auch in der nicht gespreizten Stellung mit einem Abstand zueinander angeordnet sein.

Es kann vorgesehen sein, dass die künstliche Intraokularlinse zumindest eine zweite Haptik aufweist. Insbesondere weist die zweite Haptik ebenfalls zumindest zwei Haptikteile auf, wie sie oben zur ersten Haptik erläutert wurden. Es kann auch vorgesehen sein, dass die künstliche Intraokularlinse zumindest drei derartige separate Haptiken aufweist, die in Umlaufrichtung um die optische Hauptachse beabstandet zueinander und äquidistant zueinander ausgebildet sind. Auch diese mögliche dritte Haptik kann in vorteilhafter Weise zumindest zwei Haptikteile aufweisen, die elastisch zueinander bewegbar sind. Insbesondere ist diese Bewegbarkeit in der Richtung der optischen Hauptachse ausgebildet.

In einer vorteilhaften Ausführung ist vorgesehen, dass in der Spreizstellung der beiden Haptikteile dieser zumindest einen Haptik diese Haptikteile an zumindest einer Stelle direkt aneinander anliegen und gegeneinander verspreizt sind. Damit wird eine sichere und stabile Spreizverbindung erzielt. Bevorzugt liegen die Haptikteile unter Spannung gegeneinander an.

Bevorzugt ist vorgesehen, dass zumindest ein Spreizelement zum Ausgestalten einer derartigen azimutalen Überlappung mit einem Hinterschnitt ausgebildet ist.

Bevorzugt ist vorgesehen, dass zumindest ein Spreizelement, bevorzugt sämtliche Spreizelemente, elastisch ausgebildet ist. Bevorzugt weist das mindestens eine Spreizelement eine Härte im Bereich von 60 bis 100 Shore A gemäß ISO 7619-1 auf. Dadurch ist eine Verlagerung eines Haptikteils mit einem solchen Spreizelement von der nicht gespreizten Stellung in die Spreizstellung mit relativ geringem Kraftaufwand möglich. Eine plastische Verformung an der Biegelinie eines solchen Haptikteiles kann damit vermieden werden.

In einer vorteilhaften Ausführung kann vorgesehen sein, dass das erste Spreizelement und das zweite Spreizelement zumindest in der Spreizstellung in radialer Richtung zur optischen Hauptachse betrachtet überlappend zueinander angeordnet sind. Insbesondere können sie auch zumindest bereichsweise direkt aneinander anliegend angeordnet sind. Eine derartige Ausgestaltung kann alternativ zu der oben genannten azimutalen Überlappung ausgebildet sein oder in Kombination dazu ausgebildet sein. Beispielsweise kann abhängig von der Geometrie einer Haptik und/oder der Größe der Intraokularlinse und/oder der Ausgestaltung eines Kapselsacks eine dann diesbezüglich individuell geeignete Spreizverbindung vorgesehen sein. Insbesondere können in dem Zusammenhang unterschiedliche Intraokularlinsen bereitgestellt sein, die mit unterschiedlichen Konfigurationen von Spreizverbindungen ausgebildet sind.

In einer vorteilhaften Ausführung ist vorgesehen, dass die zumindest zwei Spreizelemente so an den zumindest zwei Haptikteilen angeordnet sind, dass sie in der nicht gespreizten Stellung der Haptikteile weder in der Richtung der optischen Hauptachse noch in Umlaufrichtung um die optische Hauptachse über beide Haptikteile überstehen. Dadurch kann eine Geometrievergrößerung und/oder eine unerwünschte Formkomplexität der Haptik vermieden werden. Einerseits ist dadurch die Einstellung der Spreizstellung einfach ermöglicht.

In einer vorteilhaften Ausführung ist vorgesehen, dass die Spreizelemente im Querschnitt keilförmig ausgebildet sind. Damit können die Spreizelemente in der Richtung der optischen Hauptachse von der nicht gespreizten Stellung in die Spreizstellung mit geringer Reibkraft aneinander vorbei bewegt werden. Ruckartige oder abrupte Überführungen können dadurch vermieden werden.

Es kann vorgesehen sein, dass die beiden Spreizelemente der beiden Haptikteile an einander zugewandten Flächenbereichen dieser beiden Haptikteile ausgebildet sind. Insbesondere können diese einstückig damit ausgebildet sein. Es kann vorgesehen sein, dass zumindest ein Spreizelement als einstückiges, zusammenhängendes und somit unterbrechungsfreies Element ausgebildet ist. Beispielsweise kann dies stangenartig oder schienenartig ausgebildet sein. Ein Spreizelement kann geradlinig ausgebildet sein. Ein Spreizelement kann mit seiner Längsachse senkrecht zur optischen Hauptachse verlaufend ausgebildet sein. Es erstreckt sich in dem Zusammenhang geradlinig und radial zur optischen Hauptachse. Es kann auch vorgesehen sein, dass ein Spreizelement in radialer Richtung betrachtet geneigt zur optischen Hauptachse angeordnet ist. Insbesondere betrifft dies eine Kontaktfläche, die in der Spreizstellung zum direkten Anliegen an dem anderen Spreizelement vorgesehen ist.

Ebenso ist es möglich, dass eines der beiden Spreizelemente aus zumindest zwei separaten Spreizteilelementen ausgebildet ist. Diese können in radialer Richtung und/oder in der Richtung der optischen Hauptachse beabstandet zueinander angeordnet sein.

Es kann auch vorgesehen sein, dass zumindest ein Spreizelement als Stift oder Zapfen ausgebildet ist. Es kann vorgesehen sein, dass ein Spreizelement in der nicht gespreizten Stellung der Haptikteile zueinander in einer Grundstellung zum Haptikteil angeordnet ist und in einer gespreizten Stellung in einer dazu unterschiedlichen Haltestellung angeordnet ist. Bei einer derartigen Ausgestaltung ist somit vorgesehen, dass ein Spreizelement relativ bewegbar zu dem Haptikteil an diesem Haptikteil einstückig ausgebildet ist. Beispielsweise kann ein Spreizelement als elastisches Federelement ausgebildet sein. Dieses elastische Federelement kann ein Federzapfen oder ein Federstift sein. Insbesondere kann sich ein derartig ausgebildetes Spreizelement in der Grundstellung in einer Aussparung des zugehörigen Haptikteils unter Vorspannung befinden. Indem das benachbarte andere Haptikteil an diesem Spreizelement anliegt, wird diese Grundstellung des Spreizelements gehalten. Wird dann das Haptikteil relativ zu dem anderen Haptikteil bewegt und die Haltefunktion für das vorgespannte Spreizelement aufgelöst, verlagert sich das Spreizelement automatisch aus seiner Grundstellung in die Haltestellung und überlappt in azimutaler Richtung und/oder radialer Richtung mit dem anderen Haptikteil der Haptik. Es ist dann die Spreizstellung ausgebildet und durch die Spreizelemente selbst gehalten.

Bei alternativen Ausführungen kann vorgesehen sein, dass auch dann, wenn ein Spreizelement aus einem oder mehreren Spreizteilelementen ausgebildet ist, diese Spreizteilelemente sowohl in der Grundstellung als auch in der Haltestellung, sich entsprechend gleich weit erstrecken und sich in die gleiche Richtung erstrecken.

Sind bei einer derartigen Ausgestaltung die Spreizelemente als Zapfen oder Stifte ausgebildet, so können die komplementären zweiten Spreizteilelemente des zweiten Spreizelements als Vertiefungen oder Mulden oder Rinnen ausgebildet sein, in die die ersten Spreizteilelemente eingreifen. Diese Vertiefungen beziehungsweise Rinnen sind zumindest an einem jeweiligen Ende durch eine Wand geschlossen. Dadurch wird beim Hinwegführen beziehungsweise Herausführen eines ersten Spreizteilelements aus einem zweiten Spreizteilelement die Spreizstellung eingestellt und mit der dann erzeugten Überlappung zwischen den beiden Spreizteilelementen die Rückführung der Haptikteile von der Spreizstellung in die nicht gespreizte Stellung gesperrt.

Gerade dann, wenn ein Spreizelement zumindest zwei Spreizteilelemente aufweist, können zumindest zwei verschiedene Spreizstellungen realisiert werden. Jede dieser individuellen diskreten Spreizstellungen kann dann abhängig von der Einstellung der Spreizverbindung individuell gehalten werden und die jeweilige Spreizstellung gesperrt sein.

In einer vorteilhaften Ausführung ist vorgesehen, dass an einer Oberseite zumindest eines Haptikteils zumindest eine Koppelerhebung oder eine Koppelvertiefung ausgebildet ist. Diese Koppelerhebung oder Koppelvertiefung ist zum Eingriff eines Verstellwerkzeugs zum Einstellen einer spezifischen Spreizstellung der Haptikteile zueinander ausgebildet. Dadurch ist es für ein medizinisches Personal einfach möglich, die Spreizstellung oder eine von möglichen Spreizstellungen definiert und schnell einzustellen. Eine derartige Koppelerhebung kann beispielsweise ein Bügel sein, der mit einem entsprechenden hakenförmigen Verstellwerkzeug eingehakt werden kann. Dadurch kann dann das Haptikteil entsprechend gegenüber dem zumindest einen weiteren Haptikteil der zumindest einen Haptik in der Richtung der optischen Hauptachse, also vertikal nach oben oder nach unten, verlagert werden oder radial in Richtung zur optischen Hauptachse hin verlagert werden, um dann die Spreizstellung definiert einzustellen. Ein Verlagern in Richtung zur optischen Hauptachse kann derart erfolgen, dass ein Haptikteil in seiner Längserstreckung komprimiert bzw. gestaucht wird. Um unerwünschte Überstände an den Haptikteilen zu vermeiden, kann in vorteilhafter Weise eine Koppelvertiefung vorgesehen sein. Diese kann beispielsweise ein Sackloch oder auch ein Durchgangsloch sein. Eine derartige Koppelvertiefung kann auch einen Hinterschnitt aufweisen. Durch diese Ausgestaltung in Form eines Hinterschnitts, der auch als Aussparung bezeichnet werden kann, kann wiederum ein spezifischer Eingriff mit einem Verstellwerkzeug erfolgen. Dadurch ist die Wahrscheinlichkeit geringer, dass ein Verstellwerkzeug von dem Haptikteil beim Einstellen einer Spreizstellung abrutscht.

Es kann vorgesehen sein, dass in der Spreizstellung der zumindest zwei Haptikteile die zweite Höhe am distalen Ende, also am radial äußeren Ende, der beiden Haptikteile ausgebildet ist. Dies kann dann vorgesehen sein, wenn die zumindest zwei Haptikteile der Haptik in radialer Richtung zur optischen Hauptachse betrachtet an ihren freien, radial äußeren Enden getrennt sind.

In einer alternativen Ausführung kann vorgesehen sein, dass die zumindest zwei Haptikteile einer betrachteten Haptik in der Spreizstellung auch an diesen radial äußeren Enden direkt aneinander anliegen.

Es kann auch vorgesehen sein, dass bei einem Ausführungsbeispiel die zumindest zwei Haptikteile als parallel zueinander orientierte, radial ausgerichtete Balken ausgebildet sind. Insbesondere sind beide derartigen Haptikteile parallel zueinander orientiert. Sie sind insbesondere geradlinig ausgebildet. Es kann vorgesehen sein, dass die beiden Haptikteile über eine Teillänge ihrer gesamten radialen Länge direkt miteinander verbunden sind. Insbesondere kann diese Teillänge ausgehend von einem Ende, welches an den Optikkörper mündet, radial nach außen gerichtet sein.

Damit ist eine Biegekante, um welche dasjenige Haptikteil, welches beim Verlagern in die Spreizstellung zu dem zumindest einen anderen Haptikteil der betrachteten Haptik verstellt wird, beabstandet zu dem Optikkörper und in der Haptik selbst ausgebildet. Unerwünschte Beeinträchtigungen des Optikkörpers bei einer derartigen Biegung und Aufrechterhaltung dieser Biegung in der Spreizstellung können dadurch vermieden werden.

In einer vorteilhaften Ausführung kann vorgesehen sein, dass ein Haptikteil als Streifen beziehungsweise Balken ausgebildet ist, der radial orientiert ist. Das zumindest eine weitere Haptikteil kann in einer Ebene senkrecht zur optischen Hauptachse betrachtet dieses Haptikteil rahmenartig umgebend ausgebildet sein. Beispielsweise kann dieses andere Haptikteil eine U-Form aufweisen und einstückig sein. Die oben dargelegten vielfältigen Ausführungsbeispiele können auch bei einer derartigen Ausgestaltung einer Haptik ausgebildet sein.

Insbesondere ist die Intraokularlinse als kapselsackimplantierte Intraokularlinse ausgebildet. Sie kann auch als Kapselsack-Implantations-Intraokularlinse bezeichnet werden. Insbesondere ist sie eine Hinterkammerlinse zum Implantieren in einen Kapselsack eines Auges. Dies bedeutet, dass die Intraokularlinse bestimmungsgemäß, insbesondere nur, zur Implantation in einen Kapselsack eines Auges vorgesehen ist.

In einer vorteilhaften Ausführung ist vorgesehen, dass eine Haptik drei Haptikteile aufweist. Diese Haptikteile können in Umlaufrichtung um die optische Hauptachse benachbart und direkt aneinander anschließend zueinander ausgebildet sein. Insbesondere ist bei einer derartigen Ausgestaltung vorgesehen, dass das in azimutaler Richtung mittlere Haptikteil bei der Einstellung der zumindest einen Spreizstellung relativ zu den beiden anderen Haptikteilen verstellt wird und jeweils, insbesondere azimutal betrachtet, mit gegenüberliegenden ersten Spreizelementen an zweiten Spreizelementen, die an den beiden anderen Haptikteilen integral ausgebildet sind, anliegen und diesbezüglich verspreizt sind.

Weitere Merkmale der Erfindung ergeben sich aus den Ansprüchen, den Figuren und der Figurenbeschreibung. Die vorstehend in der Beschreibung genannten Merkmale und Merkmalskombinationen, sowie die nachfolgend in der Figurenbeschreibung genannten und/oder in den Figuren alleine gezeigten Merkmale und Merkmalskombinationen sind nicht nur in der jeweils angegebenen Kombination, sondern auch in anderen Kombinationen verwendbar, ohne den Rahmen der Erfindung zu verlassen. Es sind somit auch Ausführungen von der Erfindung als umfasst und offenbart anzusehen, die in den Figuren nicht explizit gezeigt und erläutert sind, jedoch durch separierte Merkmalskombinationen aus den erläuterten Ausführungen hervorgehen und erzeugbar sind. Es sind auch Ausführungen und Merkmalskombinationen als offenbart anzusehen, die somit nicht alle Merkmale eines ursprünglich formulierten unabhängigen Anspruchs aufweisen. Es sind darüber hinaus Ausführungen und Merkmalskombinationen, insbesondere durch die oben dargelegten Ausführungen, als offenbart anzusehen, die über die in den Rückbezügen der Ansprüche dargelegten Merkmalskombinationen hinausgehen oder abweichen.

Die in dieser Schrift angegebenen konkreten Werte von Parametern und Angaben zu Verhältnissen von Parametern bzw. Parameterwerten zur Definition von Ausführungsbeispielen der Augenlinse sind auch im Rahmen von Abweichungen, beispielsweise aufgrund von Messfehlern, Systemfehlern, DIN-Toleranzen etc. als vom Rahmen der Erfindung mit umfasst anzusehen, wodurch auch Erläuterungen, die sich auf im Wesentlichen entsprechende Werte und Angaben beziehen darunter zu verstehen sind.

Ausführungsbeispiele der Erfindung werden nachfolgend anhand schematischer Zeichnungen näher erläutert. Es zeigen:
- Fig. 1: eine schematische perspektivische Darstellung eines Ausführungsbeispiels einer künstlichen Intraokularlinse;
- Fig. 2a: eine Vorderansicht von einer Haptik einer Intraokularlinse gemäß Fig. 1 in einer nicht gespreizten Stellung von Haptikteilen dieser Haptik;
- Fig. 2b: eine Darstellung gemäß Fig. 2a, in welcher eine Spreizstellung der Haptikteile zueinander dargestellt ist;
- Fig. 3a: eine Vorderansicht von einer Haptik einer Intraokularlinse gemäß einer weiteren Ausführungsform in einer nicht gespreizten Stellung von Haptikteilen dieser Haptik;
- Fig. 3b: die Darstellung der Haptikteile gemäß Fig. 3a, wobei in Fig. 3b eine Spreizstellung der Haptikteile gezeigt ist;
- Fig. 4a: eine Vorderansicht von einer Haptik einer Intraokularlinse gemäß einer weiteren Ausführungsform in einer nicht gespreizten Stellung von Haptikteilen dieser Haptik;
- Fig. 4b: eine Darstellung der Haptikteile gemäß Fig. 4a, wobei in Fig. 4b eine erste Spreizstellung der Haptikteile gezeigt ist;
- Fig. 4c: eine Darstellung der Haptikteile gemäß Fig. 4a und Fig. 4b, wobei in Fig. 4c eine zweite Spreizstellung der Haptikteile gezeigt ist;
- Fig. 5: eine Draufsicht auf eine Haptik einer Intraokularlinse gemäß einer weiteren Ausführungsform;
- Fig. 6: eine Draufsicht auf eine Haptik einer Intraokularlinse gemäß einer weiteren Ausführungsform;
- Fig. 7: eine Schnittdarstellung der Intraokularlinse mit einer Haptik gemäß Fig. 5;
- Fig. 8: eine Seitenansicht auf einen Teilbereich der Intraokularlinse gemäß Fig. 7, in welcher die Haptikteile in einer Spreizstellung gezeigt sind;
- Fig. 9: eine Querschnittsansicht durch einen Kapselsack mit einer implantierten Intraokularlinse, welche eine Haptik wie in Fig. 8 dargestellt zeigt;
- Fig. 10: eine Querschnittsansicht durch einen Kapselsack mit einer implantierten Intraokularlinse, welche eine Haptik wie in Fig. 6 dargestellt zeigt;
- Fig. 11: eine Schnittdarstellung einer Intraokularlinse mit einer Haptik gemäß einer weiteren Ausführungsform;
- Fig. 12: eine Seitenansicht der Intraokularlinse gemäß Fig. 11, in welcher die Haptikteile in einer Spreizstellung gezeigt sind;
- Fig. 13: eine schematische Darstellung eines weiteren Ausführungsbeispiels einer Intraokularlinse mit einer Haptik in einer Draufsicht, wobei sich die Haptikteile in einer nicht gespreizten Stellung befinden;
- Fig. 14: eine schematische Darstellung eines weiteren Ausführungsbeispiels einer Intraokularlinse mit einer Haptik in einer Draufsicht, in welcher sich die Haptikteile in einer Spreizstellung befinden;
- Fig. 15: eine Darstellung eines weiteren Ausführungsbeispiels einer Intraokularlinse mit einer Haptik in einer Draufsicht;
- Fig. 16a: eine Schnittdarstellung durch eine Haptik einer Intraokularlinse gemäß Fig. 15, wobei sich die Haptikteile in einer nicht gespreizten Stellung befinden;
- Fig. 16b: eine Seitenansicht einer Haptik einer Intraokularlinse gemäß Fig. 15, wobei sich die Haptikteile in einer Spreizstellung befinden;

### Bevorzugte Ausführungsbeispiele der Erfindung

In den Figuren werden gleiche oder funktionsgleiche Elemente mit den gleichen Bezugszeichen versehen.

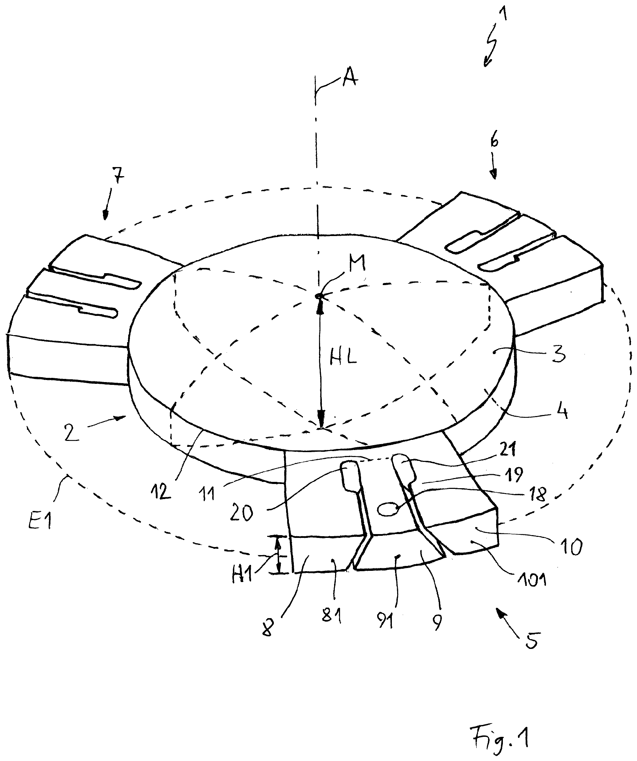

In Fig. 1 ist in einer perspektivischen Darstellung ein Ausführungsbeispiel einer künstlichen Intraokularlinse 1 gezeigt. Diese Intraokularlinse 1 ist eine Hinterkammerlinse zum Implantieren in einen Kapselsack eines Auges. Sie kann daher auch als Kapselsack-Implantations-Intraokularlinse bezeichnet werden. Die Intraokularlinse 1 weist einen Optikkörper 2 in Form einer Linse auf. Er ist zur Erzeugung einer definierten optischen Abbildungseigenschaft der Intraokularlinse 1 ausgebildet. Die Intraokularlinse 1 weist eine optische Achse bzw. eine optische Hauptachse A auf, welche eine Vorderseite 3 des Optikkörpers 2 und eine Rückseite 4 des Optikkörpers 2 durchdringt. Bei dieser Ausführungsform durchdringt die optische Hauptachse A den Optikkörper 2 an seiner geometrischen Mitte M. Die Intraokularlinse 1 weist darüber hinaus eine erste Haptik 5 auf, die sich in radialer Richtung von der optischen Hauptachse A aus betrachtet an den Optikkörper 2 anschließt. Im Ausführungsbeispiel weist die Intraokularlinse 1 darüber hinaus eine zweite Haptik 6 und eine dritte Haptik 7 auf. Die hier beispielsweise ausgebildeten drei Haptiken 5 bis 7 sind in Umlaufrichtung um die optische Hauptachse A beabstandet zueinander ausgebildet und insbesondere äquidistant zueinander angeordnet. Mit den Haptiken 5 bis 7 wird die Intraokularlinse 1 in einem Kapselsack gehalten.

Im Ausführungsbeispiel ist vorgesehen, dass die hier zumindest zwei, insbesondere drei, Haptiken 5 bis 7 vorzugsweise gleich ausgebildet sind. Nachfolgende Erläuterung anhand der ersten Haptik 5 gilt daher insbesondere auch für die hier beispielsweise vorgesehenen weiteren Haptiken 6 und 7.

Diese Haptik 5 weist im Ausführungsbeispiel ein erstes Haptikteil 8 auf. Darüber hinaus weist sie ein zweites Haptikteil 9 auf. Im Ausführungsbeispiel ist vorgesehen, dass zusätzlich ein drittes Haptikteil 10 zu der ersten Haptik 5 zugehörig ist. Die hier drei Haptikteile 8 bis 10 sind in azimutaler Richtung zur optischen Hauptachse A benachbart und unmittelbar aneinander angrenzend angeordnet. Es wird daher ein Ensemble von mehreren Haptikteilen 8 bis 10 angeordnet, welches zusammen die erste Haptik 5 bildet. Im Ausführungsbeispiel sind die drei Haptikteile 8 bis 10 als sich in radialer Richtung erstreckende Balken ausgebildet. Sie weisen vorzugsweise die gleiche radiale Erstreckung auf. Insbesondere sind sie mit ihren Längsachsen, die radial zu optischen Hauptachse A orientiert sind, in die gleiche Richtung strahlenartig nach außen orientiert. In der in Fig. 1 gezeigten nicht gespreizten Stellung der Haptikteile 8 bis 10 zueinander sind diese Längsachsen in einer gemeinsamen Ebene E1 orientiert. Diese Ebene E1 ist senkrecht zur optischen Hauptachse A orientiert. Die drei Haptikteile 8 bis 10 sind über zumindest eine Teillänge ihrer radialen Erstreckung voneinander getrennt und können sich somit relativ zueinander bewegen. Insbesondere ist zumindest das zweite Haptikteil 9 elastisch verformbar ausgebildet. Im Ausführungsbeispiel ist dazu vorgesehen, dass das zweite Haptikteil 9 in der Richtung der optischen Hauptachse A betrachtet relativ zu den anderen beiden Haptikteilen 8 und 10 bewegbar ist. Insbesondere kann es um eine Biegeline 11, die in der ersten Haptik 5 ausgebildet ist, relativ zu den anderen beiden Haptikteilen 8 und 10 in der Richtung der optischen Hauptachse A, also in die vertikale Richtung und insbesondere vertikal nach oben gebogen werden. Diese Biegelinie 11 verläuft in Umlaufrichtung um die optische Hauptachse A. Insbesondere ist diese Biegelinie 11 radial beabstandet zu einem Rand 12 des Optikkörpers 2 und verläuft somit innerhalb der ersten Haptik 5.

Die erste Haptik 5 ist so ausgebildet, dass die Haptikteile 8 bis 10 in der Richtung der optischen Hauptachse A verlagerbar sind, insbesondere in zumindest zwei voneinander verschiedene Stellungen. In der in Fig. 1 gezeigten nicht gespreizten Stellung besitzt die erste Haptik bzw. besitzen die Haptikteile 8 bis 10 maximal eine erste Höhe H1, die in der Richtung der optischen Hauptachse A bemessen ist. Diese erste Höhe H1 ist so zu verstehen, dass sie an der Stelle der ersten Haptik 5 gemessen ist, an der die erste Haptik 5 in dieser nicht gespreizten Stellung die maximale Höhe aufweist. Im Ausführungsbeispiel ist vorgesehen, dass die erste Haptik 5 in dieser nicht gespreizten Stellung plattenartig ausgebildet ist und entlang ihres gesamten Querschnittes diese erste Höhe H1 aufweist.

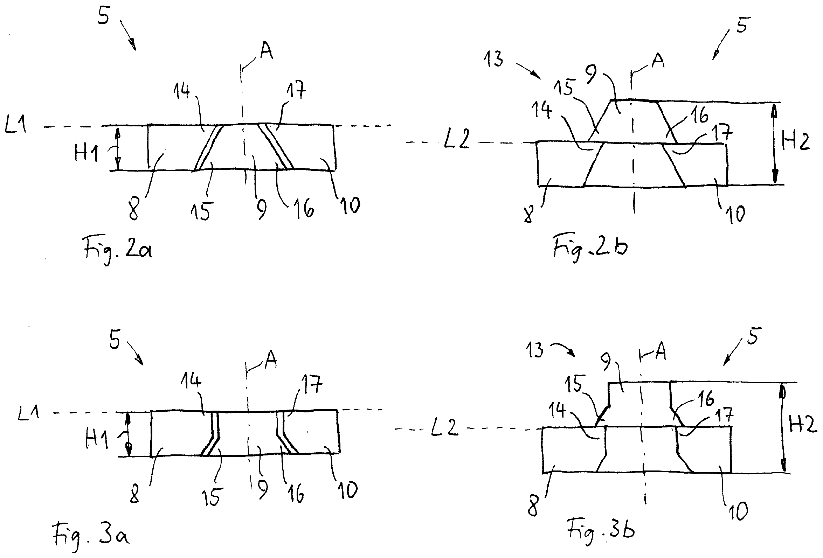

Gemäß der Erfindung ist die Intraokularlinse derart ausgebildet, dass das zweite Haptikteil relativ zum ersten Haptikteil 8 von einer nicht gespreizten Stellung in eine Spreizstellung verlagerbar ist. In dieser zumindest einen Spreizstellung weist diese erste Haptik 5 eine zweite Höhe H2 auf. Diese zweite Höhe H2, wie sie dann zu Fig. 2b und weiteren Ausführungsbeispielen nachfolgend erläutert wird, ist größer als die erste Höhe H1. Diese zweite Höhe H2 der ersten Haptik 5 ist wiederum an derjenigen Stelle der ersten Haptik 5 gemessen, an der die erste Haptik 5 in der Richtung der optischen Hauptachse A gemessen in dieser Spreizstellung eine maximale Höhe erreicht. Diese Position kann abhängig von der Ausgestaltung der Haptikteile 8 bis 10 und/oder der Ausgestaltung der Spreizverbindung 13 örtlich an einer Haptik variieren. Dies ist jedoch nachrangig für die Funktionalität. Wesentlich ist, dass an zumindest einer Stelle einer Haptik eine derartige zweite Höhe in der Spreizstellung ausgebildet ist. Besonders vorteilhaft ist es, wenn bei einer implantierten Intraokularlinse 1 die zweite Höhe H2 größer als eine maximale Dicke, zum Beispiel einer Mittendicke HL, des Optikkörpers 2 ist. Dadurch ist es ermöglicht, dass eine in einem Kapselsack implantierte Intraokularlinse 1 mit ihrem Optikkörper 2 eine Kapselsackwand nicht berührt. Insbesondere die Rückseite 4 des Optikkörpers 2 der Intraokularlinse 1 kann dadurch beabstandet zu einer hinteren Kapselsackwand angeordnet werden.

In Fig. 2a ist in einer schematischen Darstellung eine Vorderansicht der ersten Haptik 5 gezeigt. Wie bei der Ausführung in Fig. 1 des Weiteren zu erkennen ist, sind die Haptikteile 8 bis 10 bis zu ihren distalen Enden 81, 91 und 101 getrennt voneinander ausgeführt.

Wie in Fig. 2a zu erkennen ist, sind die Haptikteile 8 bis 10 so angeordnet, dass sie in einer Draufsicht überlappen. Im gezeigten Ausführungsbeispiel weist das erste Haptikteil 8 ein erstes Spreizelement 14 auf. Es ist an der Seitenwand des ersten Haptikteils 8 integriert ausgebildet, welche dem zweiten Haptikteil 9 zugewandt ist. Wie in Fig. 2a zu erkennen ist, ist bei dieser Ausführung dieses erste Spreizelement 14 im Querschnitt keilförmig gestaltet. Es erstreckt sich insbesondere über die gesamte Höhe des ersten Haptikteils 8. Diese Höhe wird in der Richtung der optischen Hauptachse A, also in vertikaler Richtung, bemessen. Des Weiteren ist zu erkennen, dass das zweite Haptikteil 9 ein zweites Spreizelement 15 aufweist. Es ist ebenfalls im Querschnitt keilförmig gebildet. Insbesondere erstreckt es sich ebenfalls über die gesamte Höhe des zweiten Haptikteils 9. Insbesondere ist es an einer Seitenwand des zweiten Haptikteils 9 integral ausgebildet, welche dem ersten Haptikteil 8 zugewandt ist. Die beiden Spreizelemente 14 und 15 sind somit komplementär geformt und in der in Fig. 2a gezeigten nicht gespreizten Stellung benachbart zueinander angeordnet, bevorzugt direkt aneinander anliegend angeordnet.

Darüber hinaus weist im gezeigten Ausführungsbeispiel das zweite Haptikteil 9 ein weiteres zweites Spreizelement 16 auf. Dieses ist an einer Seitenwand integral ausgebildet, welches dem dritten Haptikteil 10 zugewandt ist. Dieses dritte Haptikteil 10 weist ein weiteres erstes Spreizelement 17 auf. Dieses ist an einer Seitenwand integral ausgebildet, welche dem zweiten Haptikteil 9 zugewandt ist. Auch diese weiteren Spreizelemente 16 und 17 sind im Querschnitt keilförmig gebildet. Sie erstrecken sich insbesondere ebenfalls über die gesamte Höhe des zweiten Haptikteils 9 und des dritten Haptikteils 10. Wie auch bei dem weiteren zweiten Spreizelement 16 und dem weiteren ersten Spreizelement 17 zu erkennen ist, überlappen sich diese in der in Fig. 2a gezeigten nicht gespreizten Stellung, wenn das erste Haptikteil 5 in einer Draufsicht betrachtet wird. Sie sind einander zugewandt und komplementär geformt. Sie liegen insbesondere auch aneinander an. Durch die in Fig. 2a gezeigte Ausgestaltung ist die azimutale Querschnittsfläche des zweiten Haptikteils 9 trapezförmig gebildet.

In Fig. 2a befindet sich das zweite Haptikteil 9 bezüglich dem ersten Haptikteil 8 in einer nicht gespreizten Stellung. Das zweite Haptikteil 9 kann daraufhin in der Richtung der optischen Hauptachse A, also in vertikaler Richtung nach oben, relativ zum ersten Haptikteil 8 in eine Spreizstellung verlagert werden. Bei der in Fig. 2b gezeigten Ausführungsform befindet sich zusätzlich das zweite Haptikteil 9 relativ zum dritten Haptikteil 10 in einer Spreizstellung. Die zweiten Spreizelemente 15 und 16 des zweiten Haptikteils 9 wurden dazu über die ersten Spreizelemente 14 und 17 verlagert. Wie in Fig. 2b zu erkennen ist, stützen sich die aneinander vorbeibewegten Spreizelemente 15 und 14 direkt aneinander ab. Dies gilt analog für die Spreizelemente 16 und 17. Sie liegen ebenfalls direkt aneinander an. Die vertikale Überlappung der Spreizelemente 14 und 15 sowie der Spreizelemente 16 und 17 führt dazu, dass die Haptikteile in dieser Spreizstellung verharren können und die Spreizstellung damit gehalten oder arretiert ist.

Wenn das zweite Haptikteil 9 über das erste Haptikteil 8 und das dritte Haptikteil 10 verlagert wird, drückt das zweite Haptikteil 9 mit dem zweiten Spreizelement 15 auf das erste Spreizelement 14 in der Richtung der optischen Hauptachse A nach unten. Gleichzeitig drückt das weitere zweite Spreizelement 16 auf das weitere erste Spreizelement 17, wobei ebenfalls eine Kraft in der Richtung der optischen Hauptachse A nach unten ausgeübt wird. Dies führt zu einer nach unten gerichteten Verlagerung des ersten Haptikteils 8 und des dritten Haptikteils 10. Im nicht gespreizten Zustand der Haptik 5 ist die Oberseite der ersten Haptik 5 im Raum in einer Lage L1 angeordnet, vgl. Fig. 2a. In der Spreizstellung ist diese Oberseite in der Richtung der optischen Hauptachse A nach unten verlagert und erreicht im Raum die Lage L2, vgl. Fig. 2b.

Mit anderen Worten: Das zweite Haptikteil 9 kann von seiner Ruhelage in eine verspannte Lage vertikal nach oben, also in der Richtung der optischen Hauptachse A, verlagert werden. Wenn es die Höhe des ersten und dritten Haptikteils 8, 10 überwunden hat, übt es eine vertikal nach unten gerichtete Kraft auf das erste Haptikteil 8 und dritte Haptikteil 10 aus. Das zweite Haptikteil 9 verbleibt in der Lage oberhalb des ersten Haptikteils 8 und dritten Haptikteils 10 unter elastischer Spannung, während gleichzeitig das erste Haptikteil 8 und das dritte Haptikteil 10 von dem zweiten Haptikteil 9 nach unten gedrückt werden. Da das erste Haptikteil 8 und dritte Haptikteil 10 und auch das zweite Haptikteil 9 aufgrund der Spreizelemente 14 bis 17 nicht in die ursprüngliche Ruhelage zurück gelangen, sondern dauerhaft in eine jeweilige neue Ebene verlagert werden, liegt ein Spreizen vor. Das erste Haptikteil 8 und das dritte Haptikteil 10 bilden daher mit dem zweiten Haptikteil 9 eine Spreizverbindung.

Diese Spreizstellung ist durch die Spreizelemente 14 bis 17 arretiert und gelangt nicht selbsttätig in die nicht gespreizte Stellung zurück. Auch in dieser Spreizstellung überlappen die Spreizelemente 14 und 15 sowie 16 und 17 in azimutaler Richtung. In Fig. 2b ist die zweite Höhe H2 eingezeichnet, sie ist im Ausführungsbeispiel doppelt so groß wie die erste Höhe H1.

Zur Bewegung des zweiten Haptikteils 9 kann in einer vorteilhaften Ausführung ein Koppelelement vorgesehen sein. Im Ausführungsbeispiel gemäß Fig. 1 ist dieses Koppelelement durch eine Koppelvertiefung 18, die in dem zweiten Haptikteil 9 ausgebildet ist, ermöglicht. In diese Koppelvertiefung 18 kann ein Verstellwerkzeug eingreifen, um dieses zweite Haptikteil 9 gemäß der Darstellung nach oben zu biegen, sodass sich die in Fig. 2b gezeigte, diskrete Spreizstellung der Haptikteile 8 bis 10 zueinander einstellen lässt.

Es kann vorgesehen sein, dass diese Spreizelemente 14 bis 17 als geradlinige Schienen ausgebildet sind. Diese können sich gemäß ihrer Keilform in radialer Richtung erstrecken. Sie können sich über ihre gesamte radiale Längserstreckung senkrecht zur optischen Hauptachse A erstrecken. Dies bedeutet, dass eine in radialer Richtung orientierte Längsachse eines Spreizelements 14 bis 17 senkrecht zur optischen Hauptachse A orientiert ist. Möglich ist jedoch auch eine Anordnung, bei welcher eine Neigung einer derartigen Längsachse eines Spreizelements 14 bis 17 zu der optischen Hauptachse A ausgebildet ist.

Es kann vorgesehen sein, dass sich ein Spreizelement 14 bis 17 im Wesentlichen über die gesamte Längserstreckung eines Haptikteils 8 bis 10 erstreckt. Vorzugsweise ist vorgesehen, dass sich ein derartiges Spreizelement 14 bis 17 nur über eine Teillänge der gesamten in radialer Richtung bemessenen Länge eines Haptikteils 8 bis 10 erstreckt. In Fig. 1 ist bei diesem Ausführungsbeispiel vorgesehen, dass sich die Spreizelemente 14 bis 17 nur über eine Teillänge erstrecken, die ausgehend von den distalen Enden 81, 91 und 101 bis beispielsweise zu einer Hälfte oder bis zu zwei Drittel der radialen Gesamtlänge der Haptikteile 8 bis 10 ausgebildet ist. Beispielsweise kann ein radial inneres Ende der Spreizelemente 14 bis 17 an der Stelle 19, wie es in Fig. 1 eingezeichnet ist, ausgebildet sein. Im Ausführungsbeispiel sind in dem Zusammenhang dann auch noch Aussparungen 20 und 21 zwischen den Haptikteilen 8 und 9 sowie 9 und 10 gebildet. Durch diese Aussparungen kann die Bewegbarkeit des zweiten Haptikteils 9 bei der Biegung um die Biegelinie 11 verbessert erfolgen und ist dann nicht durch sich darin hinein erstreckende Spreizelemente 14 bis 17 blockiert.

In Fig. 3a ist in einer entsprechenden Vorderansicht wie in Fig. 2a ein weiteres Ausführungsbeispiel einer Intraokularlinse 1 mit einer ersten Haptik 5 gezeigt. Im Unterschied zur Darstellung gemäß Fig. 2a erstrecken sich hier die zweiten Spreizelemente 15 und 16 nicht über die gesamte Höhe des zweiten Haptikteils 9. Insbesondere sind hier zumindest Teilbereiche der einander zugewandten Seitenwände der Haptikteile 8 und 9 sowie 9 und 10 vertikal orientiert. In Fig. 3b ist in einer entsprechenden Schnittdarstellung wiederum analog zu Fig. 2a die Spreizstellung der Haptikteile 8 bis 10 zueinander gezeigt.

Bei den in Fig. 2a bis 3b gezeigten Ausführungsbeispielen ist jeweils nur eine Spreizstellung möglich.

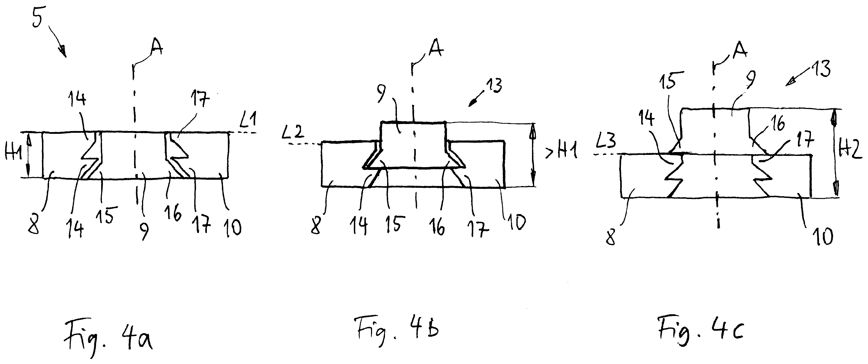

In Fig. 4a ist in einer Vorderansicht entsprechend Fig. 2a und Fig. 3a ein Ausführungsbeispiel gezeigt, bei welchem das zweite Haptikteil 9 relativ zum ersten Haptikteil 8 in zumindest zwei diskrete unterschiedliche Spreizstellungen verlagerbar ist. In Fig. 4a ist die nicht gespreizte Stellung der Haptikteile 8 bis 10 zueinander dargestellt. In Fig. 4b ist eine erste Spreizstellung des zweiten Haptikteils 9 relativ zum ersten Haptikteil 8 gezeigt. In dieser Spreizstellung sind die zweiten Spreizelemente 15 und 16 des zweiten Haptikteils 9 in nutartige Aussparungen in den Seitenwänden der seitlichen Haptikteile 8 und 9 eingreifend angeordnet. Dadurch ist das zweite Haptikteil 9 relativ zum ersten Haptikteil 8 in dieser ersten Spreizstellung wiederum gehalten und arretiert. Bei diesem Ausführungsbeispiel weist das erste Haptikteil 8 zwei separate erste Spreizelemente 14 auf. Dies betrifft ein oberes und ein unteres erstes Spreizelement 14. Entsprechend sind auch zwei separate erste Spreizelemente 17 in dem dritten Haptikteil 10 ausgebildet. Insbesondere sind diese ersten Spreizelemente 14 in der Richtung der optischen Hauptachse 8 betrachtet übereinander angeordnet und somit axial gestapelt ausgebildet. Entsprechend sind die ersten Spreizelemente 17 in dem dritten Haptikteil 10 ausgebildet. Auch hier sind die jeweiligen Spreizelemente 14, 15, 16 und 17 einstückig mit den jeweiligen Haptikteilen 8, 9 und 10 ausgebildet.

In der Darstellung gemäß Fig. 4c ist die erzeugbare zweite Spreizstellung gezeigt. Bei dieser zweiten Spreizstellung wird am distalen Ende der Haptikteile 8, 9 und 10 eine Höhe H2 erreicht, welche das Zweifache der ersten Höhe H1 ist. In der ersten Spreizstellung hingegen ist eine Höhe der ersten Haptik 5 in der Richtung der optischen Hauptachse A in der Schnittebene ausgebildet, die auch größer als die erste Höhe H1 ist, jedoch kleiner als die Höhe H2 gemäß Fig. 4c ist.

Im nicht gespreizten Zustand der Haptik 5 ist die Oberseite der ersten Haptik 5 im Raum in einer ersten Lage L1 angeordnet, vgl. Fig. 4a. In der ersten Spreizstellung ist diese Oberseite in der Richtung der optischen Hauptachse A nach unten verlagert und erreicht im Raum eine zweite Lage L2, vgl. Fig. 4b. In der zweiten Spreizstellung wird von dem zweiten Haptikteil 9 eine noch stärkere Druckkraft in der Richtung der optischen Hauptachse A auf das erste Haptikteil 8 und das dritte Haptikteil 10 ausgeübt, sodass die Oberseite der ersten Haptik 5 in eine dritte Lage L3 gelangt, welche noch tiefer als die Lage L2 ist, vgl. Fig. 4c.

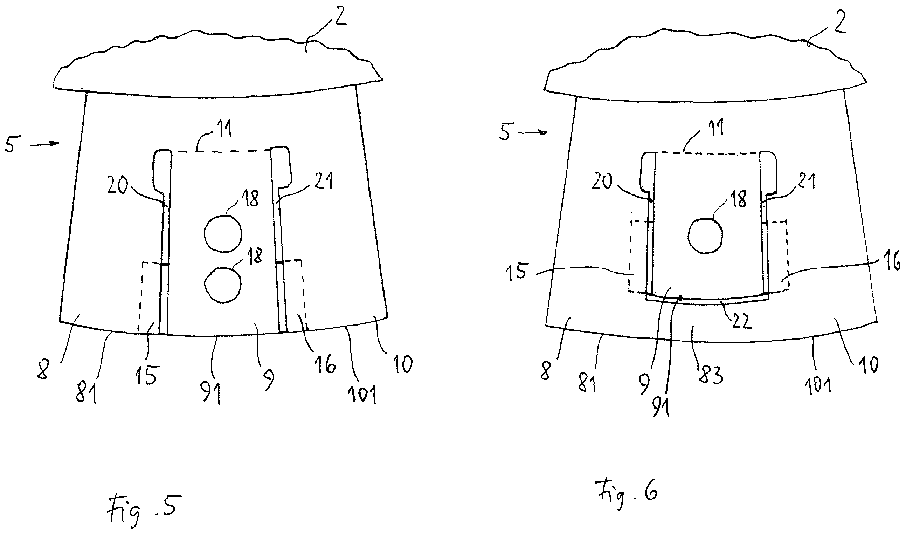

In Fig. 5 ist eine Draufsicht auf die erste Haptik 5 der Intraokularlinse 1 gemäß einer weiteren Ausführungsform gezeigt. Es ist daher eine Darstellung mit Blick in der Richtung der optischen Hauptachse A dargestellt. In dieser Ausführung ist zu erkennen, dass zwischen den Haptikteilen 8 bis 10 jeweils Aussparungen 20 und 21 vorgesehen sind, sodass sie entlang dieser Erstreckung der Aussparungen 20 und 21 nicht zusammenhängen. Die Haptikteile 8 bis 10 sind somit Biegebalken, die an ihren jeweiligen distalen Enden 81, 91 und 101 mindestens in vertikaler Richtung verlagerbar sind.

In Fig. 5 sind zwei Koppelvertiefungen 18 gezeigt. Diese können auch bei allen anderen Ausführungsformen ausgebildet sein. Damit kann in radialer Richtung an unterschiedlichen Positionen eine entsprechende Kopplung mit einem Verstellwerkzeug erfolgen. Ebenso ist es hier möglich, dass mit einem Verstellwerkzeug gleichzeitig in beide Koppelvertiefungen eingegriffen werden kann, sodass hier eine verbesserte mechanische Kopplung erreicht ist und eine entsprechende Verstellbewegung ermöglicht ist. Die Spreizelemente 14 bis 17 sind hier gestrichelt angedeutet.

Es kann auch vorgesehen sein, dass sich die zweiten Spreizelemente 15 bis 16 bis zu dem distalen Ende 91 erstrecken oder zurückversetzt dazu enden. Es kann vorgesehen sein, dass die ersten Spreizelemente 14 und 17 bis zu den distalen Enden 81 und 101 ausgebildet sind oder radial zurückversetzt dazu ausgebildet sind.

In Fig. 6 ist ein weiteres Ausführungsbeispiel einer Intraokularlinse 1 mit einer ersten Haptik 5 gezeigt. Die Darstellung ist entsprechend wie in Fig. 5, sodass auch hier in Richtung der optischen Hauptachse A auf die erste Haptik 5 geblickt wird. Bei dieser Ausführung weist die erste Haptik 5 ein erstes Haptikteil 8, ein zweites Haptikteil 10 und einen diese beiden Haptikteile 8 und 10 verbindenden Steg 83 auf. Zudem weist die erste Haptik 5 im Mittenbereich auch ein zweites Haptikteil 9 auf, welches durch Aussparungen 20, 21 und 22 von dem ersten Haptikteil 8, dem Steg 83 und dem dritten Haptikteil 10 beabstandet ist, welche einen Rahmen um das zweite Haptikteil 9 bilden. Bei dieser Ausführung ist in der Ebene senkrecht zur optischen Hauptachse A betrachtet das zweite Haptikteil 9 wiederum als sich radial erstreckender Balken beziehungsweise als sich radial erstreckende Platte ausgebildet. Insbesondere ragt das erste Haptikteil 8 zusammen mit dem dritten Haptikteil 10 und dem Steg 83 weiter radial nach außen als das zweite Haptikteil 9. Die zu den Beispielen gemäß Fig. 2a bis 5 erläuterten Möglichkeiten der Ausgestaltung der Intraokularlinse 1 können auch bei der Ausführung gemäß Fig. 6 vorgesehen sein, bis auf die Tatsache, dass sich die Spreizelemente 14 bis 17 nicht bis zu den distalen Enden 81 und 101 erstrecken können.

Bereits an dieser Stelle sei erwähnt, dass zusätzlich oder anstatt dazu auch Ausführungsbeispiele möglich sind, bei denen die Spreizelemente in radialer Richtung überlappend ausgebildet sind. Insbesondere kann auch dies sowohl in der nicht gespreizten Stellung als auch in zumindest einer möglichen Spreizstellung vorgesehen sein.

Ebenso sind Ausführungsbeispiele möglich, bei denen die Spreizelemente sowohl in azimutaler Richtung als auch in radialer Richtung überlappend ausgebildet sind.

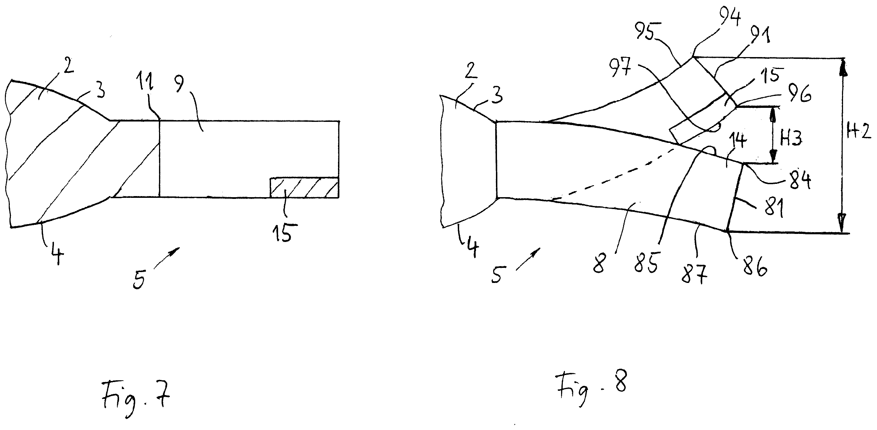

In Fig. 7 ist in einer Vertikalschnittdarstellung durch ein Ausführungsbeispiel einer Intraokularlinse 1 gemäß Fig. 5 gezeigt, in welcher sich das erste Haptikteil 8 und das zweite Haptikteil 9 in einer nicht gespreizten Stellung befinden.

In Fig. 8 ist eine Seitenansicht auf das Ausführungsbeispiel der Intraokularlinse 1 gemäß Fig. 7 gezeigt, jedoch befinden sich das zweite Haptikteil 9 relativ zum ersten Haptikteil 8 in einer Spreizstellung. Das erste Haptikteil 8 weist eine erste Kantenlinie 84 auf, welche sich an der Oberseite 85 am distalen Ende 81 des ersten Haptikteils 8 befindet. Ferner weist das erste Haptikteil 8 eine zweite Kantenlinie 86 auf, welche sich an der Unterseite 87 am distalen Ende 81 des ersten Haptikteils 8 befindet. Analog dazu weist das zweite Haptikteil 9 eine dritte Kantenlinie 94 auf, welche sich an der Oberseite 95 am distalen Ende 91 des zweiten Haptikteils 9 befindet. Eine vierte Kantenlinie 96 ist an der Unterseite 97 am distalen Ende 91 des zweiten Haptikteils 9 gebildet. Die erste Kantenlinie 84 besitzt von der vierten Kantenlinie 96 einen axialen Abstand H3 bzw. einen Abstand H3, der in der Richtung der optischen Hauptachse A gemessen ist.

Diese Ausführungsform ist besonders vorteilhaft, da sie zu einer starken Spreizung zwischen dem zweiten Haptikteil 9 und dem ersten Haptikteil 8 bzw. dem zweiten Haptikteil 9 und dem dritten Haptikteil 10 führt. Zwischen der dritten Kantenlinie 94 des zweiten Haptikteils 9 und der zweiten Kantenlinie 86 des ersten Haptikteils 8 wird eine Höhe H2 erreicht, die größer als das Zweifache der Höhe H1 ist.

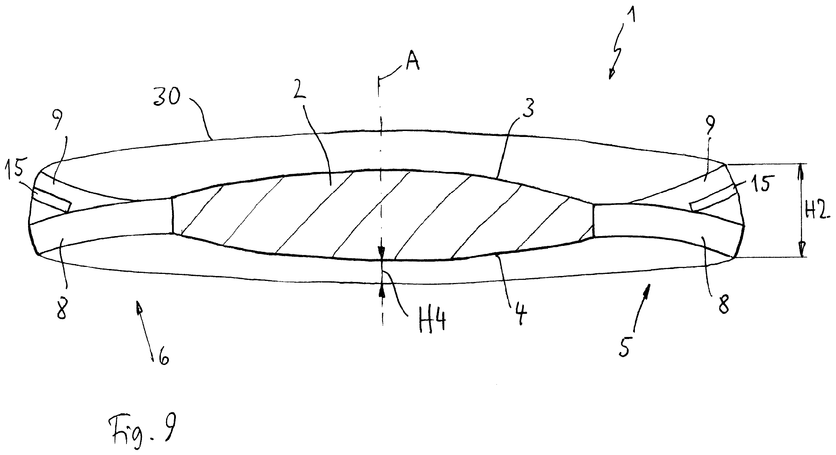

Fig. 9 zeigt in einem Querschnitt durch einen Kapselsack 30 eine Intraokularlinse 1 gemäß der in Fig. 8 dargestellten Ausführungsform mit einem ersten Haptikteil 8 und einem zweiten Haptikteil 9 von jeweils einer ersten Haptik 5 und einer zweiten Haptik 6. Es ist erkennbar, dass ein relativ großer Abstand H4 zwischen dem Optikkörper 2 und dem Kapselsack 30 erreichbar ist.

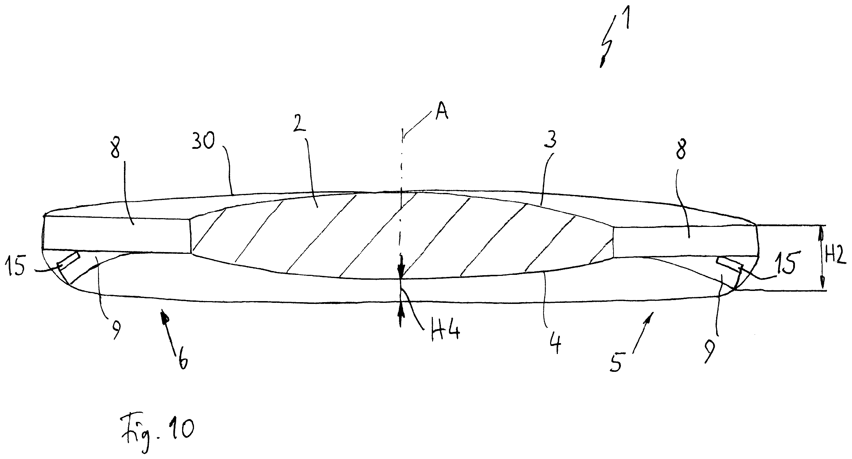

In Fig. 10 ist in einem Querschnitt durch einen Kapselsack eine Intraokularlinse 1 gemäß einer weiteren Ausführungsform dargestellt. Das erste Haptikteil 8 ist dabei so ausgeführt, dass es im Vergleich zum zweiten Haptikteil 9 eine relativ hohe Biegesteifigkeit aufweist. Das zweite Haptikteil 9 mit dem zweiten Spreizelement 15 wird bei einer Verlagerung in der Richtung der optischen Hauptachse A somit deutlich nach unten durchgebogen, wohingegen das erste Haptikteil 8 nahezu ohne Durchbiegung in seiner Lage verbleibt. Dies kann bereits ausreichen, um einen signifikanten Abstand H4 zwischen der hinteren Wand des Kapselsackes 30 und der Rückseite 4 des Optikkörpers 2 sicherzustellen, sodass dieser Bereich von Kammerflüssigkeit umspült und die Bildung von Nachstar verringert werden kann.

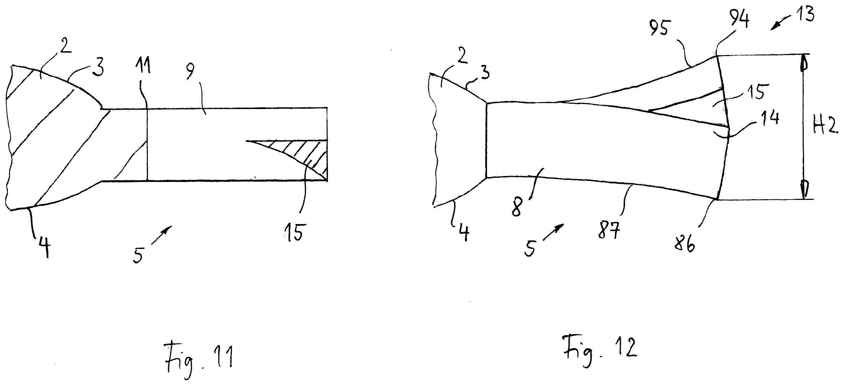

In Fig. 11 ist eine weitere Ausführungsform einer Intraokularlinse 1 mit einer ersten Haptik 5 in einem Vertikalschnitt dargestellt. Das zweite Haptikteil 9 weist ein zweites Spreizelement 15 auf, welches an der Unterseite in einer Erstreckungsrichtung, die in einer Ebene senkrecht zur optischen Hauptachse A verläuft, gekrümmt ausgebildet ist. Wenn das zweite Haptikteil 9 in der Richtung der optischen Hauptachse A nach oben bewegt wird, kann die Unterseite des zweiten Spreizelementes 15 auf der Oberseite des ersten Spreizelementes 14 flächig zur Anlage kommen. Das zweite Spreizelement 15 übt dann in einem Randbereich des ersten Haptikteils 8 auf dieses erste Haptikteil 8 eine nach unten gerichtete Druckkraft aus, was zu einer Durchbiegung des ersten Haptikteils 8 führt, vgl. Fig. 12. Dies gilt analog auch für das weitere zweite Spreizelement 16, dessen gekrümmte Unterseite die Oberfläche des Weiteren ersten Spreizelementes 17 flächig berührt. Dadurch ergibt sich zwischen der dritten Kantenlinie 94 des zweiten Haptikteils 9 und der zweiten Kantenlinie 86 des ersten Haptikteils 8 eine Höhe H2, welche nahezu den doppelten Betrag von der Höhe H1 erreicht. Diese Ausführungsform ist vorteilhaft, da durch den großen Flächenkontakt zwischen dem zweiten Spreizelement 15 und der Oberseite 85 des ersten Haptikteils 8 eine sehr stabile Spreizverbindung 13 erzielt wird.

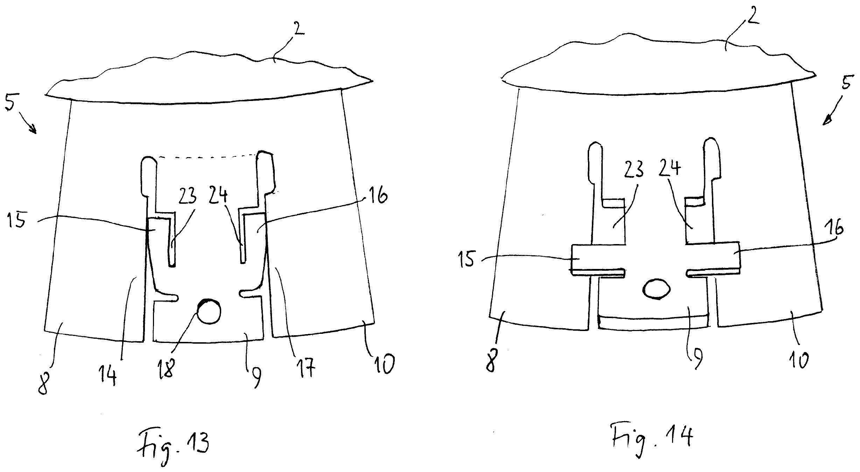

In Fig. 13 ist in einer schematischen Darstellung ein weiteres Ausführungsbeispiel einer Intraokularlinse mit einer ersten Haptik 5 in einer Draufsicht gezeigt. Bei dieser Ausführungsform ist zumindest ein zweites Spreizelement 15 und ein weiteres zweites Spreizelement 16 vorgesehen. Die zweiten Spreizelemente 15 und 16 sind einstückig mit dem zweiten Haptikteil 9 ausgebildet. Bei diesem Ausführungsbeispiel sind die ersten Spreizelemente 14 und 17 durch die Haptikteile 8 und 10 gebildet. In der in Fig. 13 gezeigten nicht gespreizten Stellung sind die zweiten Spreizelemente 15 und 16 in einer vorgespannten Lage dargestellt. In dieser Lage sind sie in Aussparungen 23 und 24, die an Seitenwänden des zweiten Haptikteils 9 vorgesehen sind, welche den Haptikteilen 8 und 10 zugewandt sind, innenliegend angeordnet. Diese vorgespannte Lage wird durch das direkte Anliegen der zweiten Spreizelemente 15 und 16 an den Haptikteilen 8 und 10 gehalten. Wird nun das zweite Haptikteil 9 bewegt, insbesondere um die Biegelinie 11 relativ zu den anderen Haptikteilen 8 und 10 in der Richtung der optischen Hauptachse A, in diesem Fall vertikal nach oben, bewegt, so wird während dieser Bewegung eine Position erreicht, bei welcher die zweiten Spreizelemente 15 und 16 nicht mehr in Kontakt mit den zugewandten Seitenwänden der Haptikteile 8 und 10 positioniert sind. In dieser Stellung schwenken die zweiten Spreizelemente 15 und 16 seitlich nach außen und liegen dann zur Halterung der ausgebildeten Spreizstellung an den ersten Spreizelementen 14 und 17 an den Oberseiten der Haptikteile 8 und 10 an. Bei dieser Ausführung ist lediglich in der Spreizstellung eine Überlappung zwischen den ersten Spreizelementen 14 und 15 sowie den zweiten Spreizelementen 16 und 17 gebildet. Die Spreizstellung ist schematisch in Fig. 14 gezeigt, in welcher eine Draufsicht auf die erste Haptik 5 dargestellt ist.

Die erste Haptik 5 wird so gefertigt, wie dies in Fig. 14 dargestellt ist, also mit seitlich vom zweiten Haptikteil 9 abstehendem zweiten Spreizelement 15 und weiterem zweiten Spreizelement 16. Vor einem Einsetzen der Intraokularlinse 1 in ein Auge wird das zweite Spreizelement 15 um -90° geschwenkt, und das weitere zweite Spreizelement 16 wird um +90° geschwenkt, sodass beide Spreizelemente 15, 16 jeweils in die zugehörigen Aussparungen 23, 24 eingreifen und dort in vorgespannter Lage verbleiben. Die Implantation der Intraokularlinse erfolgt dann mit den Spreizelementen 15, 16 in der vorgespannten Lage. Wenn sich die Intraokularlinse 1 im Kapselsack befindet, verlagert ein Operateur das zweite Haptikteil 9 in der Richtung der optischen Hauptachse A und ermöglicht ein Verschwenken der zweiten Spreizelemente 15 und 16, sodass eine Spreizstellung erreicht wird.

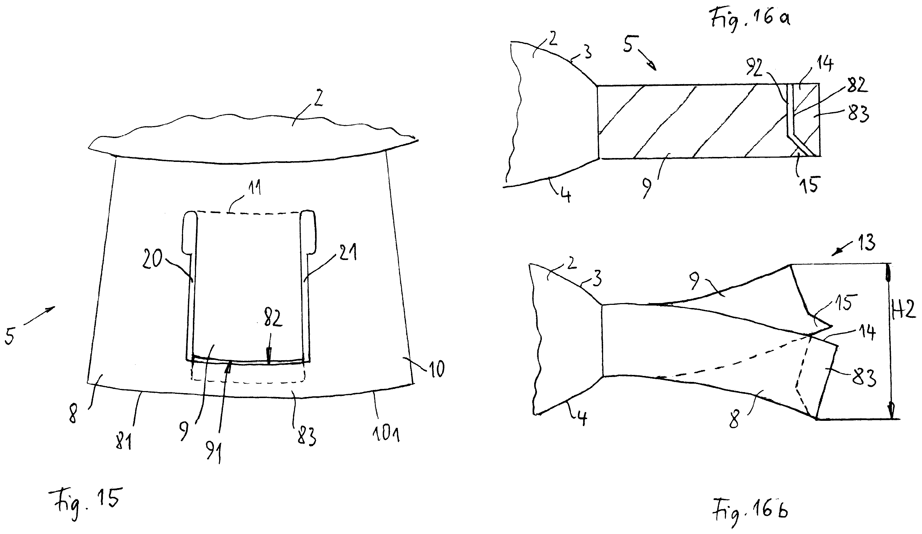

In Fig. 15 ist in einer schematischen Draufsicht ein weiteres Ausführungsbeispiel für eine Intraokularlinse 1 mit einer ersten Haptik 5 gezeigt. Diese ist vorzugsweise entsprechend der Ausführung in Fig. 6 gezeigt, bei welcher das erste Haptikteil 8 mittels eines Steges 83 mit dem dritten Haptikteil 10 rahmenartig verbunden ist. Die Baugruppe aus erstem Haptikteil 8, Steg 83 und drittem Haptikteil 10 weist eine relativ hohe Biegesteifigkeit auf. Der Steg 83 gilt als ein Teil des ersten Haptikteils 8. Das erste Haptikteil 8 weist ein erstes Spreizelement 14 auf, und das zweite Haptikteil 9 weist ein zweites Spreizelement 15 auf, vgl. Fig. 16a. Das zweite Spreizelement 15 ist an einem distalen Ende 91 des zweiten Haptikteils 9 angeordnet und bildet eine Verlängerung dieses distalen Endes 91, wobei das distale Ende 91 bevorzugt eine vertikal angeordnete Stirnfläche 92 aufweist. Bevorzugt verläuft das zweite Spreizelement 15 im Querschnitt geneigt zu der Stirnfläche 92. Das erste Haptikteil 8 weist ein proximales Ende 82 auf, welches komplementär und in einem engen Abstand zu dem distalen Ende 91 mit dem zweiten Spreizelement 15 angeordnet ist.

Sämtliche Komponenten der Intraokularlinse 1 sind aus einem Werkstoff gebildet, welcher eine Härte im Bereich von 60 bis 100 Shore A gemäß ISO 7619-1 besitzt, die Komponenten sind also relativ weich und elastisch verformbar. Dies gilt für sämtliche Ausführungsformen, die in dieser Schrift offenbart sind.

Wird das zweite Haptikteil 9 entlang der Richtung der optischen Hauptachse A nach oben verlagert, stößt das zweite Spreizelement 15 an den Steg 83 an. Aufgrund der geringen Härte des zweiten Haptikteils 9 wird dieses dabei radial gestaucht und kann über den Steg 83 verlagert werden, bis das zweite Spreizelement 15 an dem ersten Spreizelement 14 zur Anlage kommt, siehe Fig. 16b.

Diese Ausführungsform ist vorteilhaft, da die Haptik 5 in dieser Spreizstellung eine Höhe H2 erreicht, welche mehr als das Zweifache der Höhe H1 ist.

### Bezugszeichen:

- 1: Intraokularlinse
- 2: Optikkörper
- 3: Vorderseite des Optikkörpers
- 4: Rückseite des Optikkörpers
- 5: erste Haptik
- 6: zweite Haptik
- 7: dritte Haptik
- 8: erstes Haptikteil
- 9: zweites Haptikteil
- 10: drittes Haptikteil
- 11: Biegelinie
- 12: Rand des Optikkörpers
- 13: Spreizverbindung
- 14: erstes Spreizelement
- 15: zweites Spreizelement
- 16: weiteres zweites Spreizelement
- 17: weiteres erstes Spreizelement
- 18: Koppelvertiefung
- 19: Stelle
- 20: Aussparung
- 21: Aussparung
- 22: Aussparung
- 23: Aussparung
- 24: Aussparung
- 30: Kapselsack
- 81: distales Ende des ersten Haptikteils
- 82: proximales Ende des ersten Haptikteils
- 83: Steg
- 84: erste Kantenlinie
- 85: Oberseite des ersten Haptikteils
- 86: zweite Kantenlinie
- 87: Unterseite des ersten Haptikteils
- 91: distales Ende des zweiten Haptikteils
- 92: Stirnfläche des zweiten Haptikteils
- 94: dritte Kantenlinie
- 95: Oberseite des zweiten Haptikteils
- 96: vierte Kantenlinie
- 97: Unterseite des zweiten Haptikteils
- 101: distales Ende des dritten Haptikteils
- A: optische Hauptachse
- E1: Ebene der ersten Haptik in nicht gespreizter Stellung
- H1: erste Höhe
- H2: zweite Höhe
- H3: Abstand
- H4: Abstand
- HL: Mittendicke
- L1: erste Lage
- L2: zweite Lage
- L3: dritte Lage
- M: geometrische Mitte

## Patentansprüche

1. Intraokularlinse (1) mit einem Optikkörper (2) und mit zumindest einer Haptik (5, 6, 7), die mit dem Optikkörper (2) gekoppelt ist, wobei die zumindest eine Haptik (5, 6, 7) ein erstes Haptikteil (8) und zumindest ein benachbart dazu ausgebildetes zweites Haptikteil (9) aufweist, welches relativ zum ersten Haptikteil (8) elastisch bewegbar ist, und mit einer optischen Hauptachse (A), die eine Vorderseite (3) und eine Rückseite (4) des Optikkörpers (2) durchdringt, wobei die Haptik (5, 6, 7) in der Richtung der optischen Hauptachse (A) gemessen maximal eine erste Höhe (H1) aufweist,
**dadurch gekennzeichnet, dass** das zweite Haptikteil (9) relativ zum ersten Haptikteil (8) von einer nicht gespreizten Stellung in eine Spreizstellung so verlagerbar ist, dass die Haptik (5, 6, 7) in der Richtung der optischen Hauptachse gemessen maximal eine zweite Höhe (H2) erreicht, welche größer als die erste Höhe (H1) ist, und in der Spreizstellung das zweite Haptikteil (9) relativ zu dem ersten Haptikteil (8) arretierbar ist, wobei das erste Haptikteil (8) zumindest ein erstes Spreizelement (14, 17) aufweist, und das zweite Haptikteil (9) zumindest ein zweites Spreizelement (15, 16) aufweist, wobei das erste Spreizelement (14, 17) und das zweite Spreizelement (15, 16) zumindest in der Spreizstellung in Umlaufrichtung um die optische Hauptachse (A) betrachtet überlappend zueinander und zumindest bereichsweise direkt aneinander anliegend angeordnet sind.

2. Intraokularlinse (1) nach Anspruch 1,
**dadurch gekennzeichnet, dass**
das erste Spreizelement (14, 17) und das zweite Spreizelement (15, 16) zumindest in der Spreizstellung in radialer Richtung von der optischen Hauptachse (A) ausgehend überlappend zueinander und zumindest bereichsweise direkt aneinander anliegend angeordnet sind.

3. Intraokularlinse (1) nach Anspruch 1 oder 2,
**dadurch gekennzeichnet, dass**
die Spreizelemente (14, 15, 16, 17) so an den Haptikteilen (8, 9) angeordnet sind, dass sie in der nicht gespreizten Stellung der Haptikteile (8, 9) weder in der Richtung der optischen Hauptachse (A) noch in Umlaufrichtung um die optische Hauptachse (A) über die beiden Haptikteile (8, 9) überstehen.

4. Intraokularlinse (1) nach einem der vorehrgehenden Ansprüche,
**dadurch gekennzeichnet, dass**
die Spreizelemente (14, 15, 16, 17) im Querschnitt keilförmig ausgebildet sind.

5. Intraokularlinse (1) nach einem der vorhergehenden Ansprüche,
**dadurch gekennzeichnet, dass**
die zweite Höhe (H2) größer als eine Mittendicke (HL) des Optikkörpers (2) ist.

6. Intraokularlinse (1) nach einem der vorhergehenden Ansprüche,
**dadurch gekennzeichnet, dass**
an einer Oberseite eines Haptikteils (8, 9) eine Koppelerhebung oder eine Koppelvertiefung (18) ausgebildet ist, welche zum Eingriff eines Verstellwerkzeugs zum Einstellen einer spezifischen Spreizstellung der Haptikteile (8, 9) zueinander ausgebildet ist.

7. Intraokularlinse (1) nach einem der vorhergehenden Ansprüche,
**dadurch gekennzeichnet, dass**
die Intraokularlinse (1) eine Hinterkammerlinse zum Implantieren in einen Kapselsack eines Auges ist.

## Claims

1. Intraocular lens (1) having an optical body (2) and having at least one haptic element (5, 6, 7) coupled to the optical body (2), wherein the at least one haptic element (5, 6, 7) has a first haptic component (8) and at least a second haptic component (9) formed adjacent thereto, which is movable elastically relative to the first haptic component (8), and having a principal optical axis (A) that penetrates through a front side (3) and a reverse side (4) of the optical body (2), wherein the haptic element (5, 6, 7), measured in the direction of the principal optical axis (A), has a maximum of a first height (H1),
**characterized in that** the second haptic component (9) is displaceable relative to the first haptic component (8) from a non-spread position to a spread position in such a way that the haptic element (5, 6, 7), measured in the direction of the principal optical axis, attains a maximum of a second height (H2) which is greater than the first height (H1), and, in the spread position, the second haptic component (9) can be locked in place relative to the first haptic component (8), wherein the first haptic component (8) has at least a first spreading element (14, 17), and the second haptic component (9) has at least a second spreading element (15, 16), wherein the first spreading element (14, 17) and the second spreading element (15, 16), viewed in circumferential direction around the principal optical axis (A), at least in the spread position, are in a mutually overlapping arrangement and directly adjacent to one another at least in some regions.

2. Intraocular lens (1) according to Claim 1,
**characterized in that**
the first spreading element (14, 17) and the second spreading element (15, 16), at least in the spread position, are arranged so as to overlap one another in radial direction proceeding from the principal optical axis (A) and directly adjacent to one another at least in some regions.

3. Intraocular lens (1) according to Claim 1 or 2,
**characterized in that**
the spreading elements (14, 15, 16, 17) are arranged on the haptic components (8, 9) such that, in the non-spread position of the haptic components (8, 9), they do not protrude beyond the two haptic components (8, 9) either in the direction of the principal optical axis (A) or in circumferential direction around the principal optical axis (A).

4. Intraocular lens (1) according to any of the preceding claims,
**characterized in that**
the spreading elements (14, 15, 16, 17) are of wedge-shaped cross section.

5. Intraocular lens (1) according to any of the preceding claims,
**characterized in that**
the second height (H2) is greater than a center thickness (HL) of the optical body (2).

6. Intraocular lens (1) according to any of the preceding claims,
**characterized in that**
a coupling elevation or coupling depression (18) formed on a top face of a haptic component (8, 9) is set up for engagement of an adjustment tool for establishment of a specific spread position of the haptic components (8, 9) relative to one another.

7. Intraocular lens (1) according to any of the preceding claims,
**characterized in that**
the intraocular lens (1) is a posterior chamber lens for implantation into a capsular bag of an eye.

## Revendications

1. Lentille intraoculaire (1) comprenant un corps d'optique (2) et au moins une haptique (5, 6, 7) qui est couplée au corps d'optique (2), l'au moins un haptique (5, 6, 7) comportant une première partie d'haptique (8) et au moins une deuxième partie d'haptique (9) qui est formée de manière adjacente à celle-ci et qui est mobile élastiquement par rapport à la première partie d'haptique (8), et comprenant un axe optique principal (A) qui passe par un côté avant (3) et un côté arrière (4) du corps d'optique (2), l'haptique (5, 6, 7) ayant une première hauteur maximale (H1) mesurée dans la direction de l'axe optique principal (A),
**caractérisé en ce que** la deuxième partie d'haptique (9) peut être déplacée par rapport à la première partie d'haptique (8) d'une position non écartée à une position écartée de sorte que l'haptique (5, 6, 7) atteint au maximum une deuxième hauteur (H2), mesurée dans la direction de l'axe optique principal, qui est supérieure à la première hauteur (H1) et, dans la position écartée, la deuxième partie d'haptique (9) peut être bloquée par rapport à la première partie d'haptique (8), la première partie d'haptique (8) comportant au moins un premier élément d'écartement (14, 17), et la deuxième partie d'haptique (9) comportant au moins un deuxième élément d'écartement (15, 16), le premier élément d'étalement (14, 17) et le deuxième élément d'étalement (15, 16) étant disposés au moins dans la position d'étalement dans la direction de rotation autour de l'axe optique principal (A) de manière à se chevaucher et à venir directement en appui l'un sur l'autre au point par zones.

2. Lentille intraoculaire (1) selon la revendication 1,
**caractérisée en ce que**
le premier élément d'écartement (14, 17) et le deuxième élément d'écartement (15, 16) sont disposés au moins dans la position d'écartement dans une direction radiale depuis l'axe optique principal (A) de manière à se chevaucher et à venir directement en appui l'un sur l'autre au point par zones.

3. Lentille intraoculaire (1) selon la revendication 1 ou 2,
**caractérisée en ce que**
les éléments d'écartement (14, 15, 16, 17) sont disposés sur les parties d'haptiques (8, 9) de sorte que dans la position non écartée des parties d'haptiques (8, 9), ils ne fassent pas saillie des deux parties d'haptique (8, 9) ni dans la direction de l'axe optique principal (A) ni dans la direction de rotation autour de l'axe optique principal (A).

4. Lentille intraoculaire (1) selon l'une des revendications précédentes,
**caractérisée en ce que**
les éléments d'écartement (14, 15, 16, 17) ont une section transversale en forme de coin.

5. Lentille intraoculaire (1) selon l'une des revendications précédentes,
**caractérisée en ce que**
la deuxième hauteur (H2) est supérieure à une épaisseur centrale (HL) du corps d'optique (2).

6. Lentille intraoculaire (1) selon l'une des revendications précédentes,
**caractérisée en ce que**
sur un côté supérieur d'une partie d'haptique (8, 9) est formée une élévation de couplage ou une dépression de couplage (18) qui est conçue pour l'engagement d'un outil de réglage destiné à régler une position d'écartement spécifique des parties d'haptique (8, 9) l'une par rapport à l'autre.

7. Lentille intraoculaire (1) selon l'une des revendications précédentes,
**caractérisée en ce que**
la lentille intraoculaire (1) est une lentille de chambre postérieure destinée à être implantée dans un sac capsulaire d'un œil.
